Europäisches Patentamt

European Patent Office

Office européen des brevets

(11) Veröffentlichungsnummer: **0 170 964**
**B1**

(12) **EUROPÄISCHE PATENTSCHRIFT**

(45) Veröffentlichungstag der Patentschrift:
**07.12.88**

(21) Anmeldenummer: **85109146.2**

(22) Anmeldetag: **22.07.85**

(51) Int. Cl.⁴: **C 07 C 53/12,** C 07 C 53/08,
C 07 C 51/12, C 07 C 51/54

(54) **Verfahren zur Herstellung von Essigsäureanhydrid und ggf. Essigsäure.**

(30) Priorität: **08.08.84 DE 3429180**

(43) Veröffentlichungstag der Anmeldung:
**12.02.86 Patentblatt 86/7**

(45) Bekanntmachung des Hinweises auf die Patenterteilung:
**07.12.88 Patentblatt 88/49**

(84) Benannte Vertragsstaaten:
**AT BE CH DE FR GB IT LI NL**

(56) Entgegenhaltungen:
**EP-A- 0 026 280**
**EP-A- 0 133 331**
**US-A- 4 270 015**

(73) Patentinhaber: **HOECHST AKTIENGESELLSCHAFT,
Postfach 80 03 20, D-6230 Frankfurt am Main 80 (DE)**

(72) Erfinder: **Erpenbach, Heinz, Dr., Oberbuschweg 22,
D-5000 Köln 50 (DE)**
Erfinder: **Gehrmann, Klaus, Dr.,
Geschwister-Scholl-Strasse 32, D-5042 Erftstadt (DE)**
Erfinder: **Hörstermann, Peter, Dr.,
Theodor-Heuss-Strasse 2, D-5042 Erftstadt (DE)**
Erfinder: **Schmitz, Klaus, Henriette-Lott-Weg 4,
D-5030 Hürth (DE)**

## Beschreibung

Die Erfindung betrifft ein Verfahren zur Herstellung von Essigsäureanhydrid und ggf. Essigsäure durch Umsetzung von Kohlenmonoxid und ggf. Wasserstoff mit Methylacetat und/oder Dimethylether, ggf. in Mischung mit Methanol und/oder Wasser, bei Temperaturen von 350 bis 575 K und Drücken von 1 bis 300 bar in Gegenwart eines Katalysatorsystems aus Edelmetallen der Gruppe VIII des Periodensystems der Elemente oder deren Verbindungen, Jod und/oder dessen Verbindungen sowie ggf. carbonylbildenden Nichtedelmetallen der Gruppen IV, V, VI, VII oder VIII des Periodensystems der Elemente oder deren Verbindungen und ggf. einer aliphatischen Carbonsäure mit 1 bis 4 Kohlenstoffatomen, welches dadurch gekennzeichnet ist, dass das Katalysatorsystem als zusätzlichen Promotor Tetrabutylphosphoniumjodid enthält.

Die DE 2 450 965 C2 betrifft ein Verfahren zur Herstellung von Essigsäureanhydrid aus Essigsäuremethylester und Kohlenmonoxid, ggf. in Anwesenheit von 5–50 Vol% Wasserstoff, bei Drücken von 1 bis 500 bar und Temperaturen von 50 bis 250°C in Gegenwart von Katalysatoren, die Edelmetalle der VIII. Gruppe des Periodensystems oder deren Verbindungen sowie Jod und/oder Jodverbindungen sowie ggf. carbonylbildende Metalle, z.B. Co, Ni oder Fe, enthalten. Als Jodverbindungen werden auch quaternäre Ammonium- oder Phosphoniumverbindungen wie Tetramethylammoniumjodid und Tetraethylphosphoniumjodid genannt. Beide Verbindungen sind jedoch für das Katalysatorsystem wenig geeignet, da sie aufgrund ihres hohen Schmelzpunktes und ihrer geringen Löslichkeit durch Ausfallen eine kontinuierliche Kreislaufführung der Katalysatorlösung verhindern. Als Katalysatorbestandteile werden zusätzlich Alkyl- oder Arylphosphine wie Tri-n-butylphosphin und Triphenylphosphin beansprucht.

Die DE 2 610 036 C2 beschreibt ein vergleichbares Verfahren zur Herstellung von Monocarbonsäureanhydriden, jedoch mit dem Unterschied, dass neben dem Edelmetall der Gruppe VIII des Periodensystems, einem Jodid sowie einer Organostickstoff- oder Organophosphorverbindung mit dreibindigem Stickstoff bzw. Phosphor ein Metall der Gruppen IVa, Va oder VIa des Periodensystems, vorzugsweise Chrom, eingesetzt wird.

Beim Verfahren der DE 2 610 036 C2 wirkt es sehr störend, dass die Metallverbindungen und Folgeprodukte des Mehrfachpromotors in siedendem Acetanhydrid weitgehend unlöslich sind, so dass die beim kontinuierlichen Verfahren erforderliche Kreislaufführung des Katalysatorsystems sehr erschwert, wenn nicht gar unmöglich gemacht wird. Ebenso beeinträchtigen diese unlöslichen Verbindungen die Trennung des Acetanhydrids vom Katalysatorsystem in unzulässigem Masse. So tritt beispielsweise bei der destillativen Aufarbeitung des Reaktionsgemisches eine Belegung des Verdampfers mit Chromsalzen auf, wodurch der Wärmeübergang empfindlich gestört ist. Die infolgedessen notwendige Steigerung der Verdampfertemperatur führt zu einer erheblichen Schädigung des Katalysatorsystems.

Die DE 2 939 839 A1 beschreibt ein Verfahren zur Herstellung von Essigsäureanhydrid in Gegenwart eines edelmetallhaltigen Katalysatorsystems, welches zusätzlich eine quaternäre Organophosphorverbindung sowie eine im Reaktionsgemisch lösliche Zirkonverbindung enthält. Dabei bestehen die quaternären Organophosphorverbindungen aus Addukten von organischen Phosphinen mit Methyljodid. Diese Addukte weisen zum Beispiel im Falle des besonders günstig gelagerten Tributylmethylphosphoniumjodids Schmelzpunkte von fast 140°C auf. Dieser Umstand erfordert zur Erhaltung der Schmelzflüssigkeit eine gleich hohe Temperatur bei der Abtrennung der Katalysatorlösung, was den Katalysator als solchen thermisch stark belastet. Eine Absenkung der Temperatur zur schonenderen Abtrennung der Katalysatorlösung, beispielsweise unter vermindertem Druck, ist aus diesen Gründen nicht ohne das Risiko einer plötzlichen Kristallisation durchführbar.

Die nicht vorveröffentlichte EP 0 133 331 A1 beschreibt die Herstellung von Essigsäure und/oder Methylacetat in der Flüssigphase durch Umsetzung von Methanol mit Kohlenmonoxid in Gegenwart eines Katalysatorsystems, bestehend aus einer löslichen Palladiumverbindung und z.B. Methyljodid, einem ionischen Halogenid und einem Sulphon oder Sulphoxid. Als ionisches Halogenid wird u.a. auch Tetrabutylphosphoniumjodid aufgezählt. Lösemittel sind im allgemeinen entbehrlich, doch können gewünschtenfalls solche eingesetzt werden, u.a. z.B. Dimethylether. Vom Verfahren der Erfindung unterscheidet sich das Verfahren der EP 0 133 331 A1 u.a. dadurch, dass der Katalysator zwingend eine organische Schwefelverbindung enthält und infolge der Umsetzung von lediglich Methanol mit Kohlenmonoxid Essigsäureanhydrid nicht hergestellt werden kann.

Durch die eingangs geschilderte Erfindung werden nun bei hervorragender Katalysatoraktivität, ausgedrückt in g erhaltenen Essigsäureanhydrids je g elementares Edelmetall und Stunde, diese Nachteile vermieden, da das Tetrabutylphosphoniumjodid mit einem Schmelzpunkt von 103°C günstigere Bedingungen für die Abtrennung der Katalysatorlösung von den Reaktionsprodukten zulässt. Dabei erweist sich überraschenderweise das Tetrabutylphosphoniumjodid unter den Reaktionsbedingungen als absolut beständig, wogegen entsprechende quaternäre Phosphoniumjodide mit $C_5$- und mehr Kohlenstoffatomen einem Zerfall unter Bildung entsprechender methylsubstituierter Phosphoniumjodide unterliegen. Der Einsatz quaternärer Phosphoniumjodide mit weniger als 4 Kohlenstoffatomen im jeweiligen Substituenten ist wegen ihrer Schmelzpunkte von 200°C und mehr unvorteilhaft.

Die Erfindung ist bevorzugt und wahlweise dadurch gekennzeichnet, dass man

a) das Katalysatorsystem Edelmetall (-verbindung)/Jod(-verbindung)/ carbonylbildendes Nichtedelmetall(-verbindung)/ Carbonsäure/Tetrabutylphosphoniumjodid im Atom- bzw. Molverhältnis 1 : (1–1400) : (0–10) : (0–2000) : (1–1200) einsetzt;

b) Methylacetat, Methanol und Wasser oder Dimethylether, Methanol und Wasser im Molverhältnis 1 : (0 bis 5) : (0 bis 1) einsetzt;

c) Kohlenmonoxid/Wasserstoffgemische mit bis zu 10 Vol% Wasserstoff einsetzt.

Bevorzugt wird das Verfahren der Erfindung bei Temperaturen von 400 bis 475 K und Drücken von 20 bis 150 bar durchgeführt. Je Mol Methylacetat und/oder Dimethylether setzt man vorzugsweise 0,0001 bis 0,01 mol der Edelmetalle der Gruppe VIII des Periodensystems der Elemente oder seiner Verbindungen ein.

Vorzugsweise setzt man das Katalysatorsystem Edelmetall(-verbindung)/Jod (-verbindung)/carbonylbildendes Nichtedelmetall(-verbindung)/Carbonsäure/Tetrabutylphosphoniumjodid im Atom- bzw. Molverhältnis 1 : (10–300) : (0–8) : (0–600) : (10–300) ein. Bei einem eventuellen Einsatz einer Carbonsäure wird Essigsäure bevorzugt.

Als Katalysator kann jedes Edelmetall der Gruppe VIII des Periodensystems (Ru, Rh, Pd, Os, Ir, Pt) eingesetzt werden. Die höchste Aktivität besitzt jedoch das Rhodium. Als Einsatzform des Rhodiums bzw. der anderen Edelmetalle können alle Verbindungen eingesetzt werden, die unter den Reaktionsbedingungen löslich sind und den aktiven Edelmetallcarbonylkomplex bilden. z.B. $RhCl_3 \cdot 3H_2O$, $[Rh(CO)_2Cl]_2$, $[Pd(CO)_2I]_2$, $IrCl_3$, $Pd(CH_3CO_2)_2$, $PdCl_2$, $Pd(C_5H_7O_2)_2$.

Als Jodverbindungen setzt man in erster Linie Methyljodid, aber auch Acetyljodid oder Jodwasserstoff ein.

Die ggf. als Promotoren verwendeten, Carbonylkomplexe bildenden Nicht-Edelmetalle der Gruppen IV, V, VI, VII und VIII werden zweckmässig in einer gut löslichen Form, z.B. als Acetylacetonat oder als Carbonyl in die Reaktion eingesetzt. Es kommen hier bevorzugt Verbindungen der Metalle Ti, Zr, V, Nb, Ta, Cr, Mo, W, Mn, Re, Fe, Co oder Ni infrage.

Die Durchführung des erfindungsgemässen Verfahrens kann diskontinuierlich wie auch kontinuierlich erfolgen und wird nachstehend beschrieben:

In einem Druckreaktor aus Hastelloy B2 wird die Reaktionsmischung unter den angegebenen Bedingungen umgesetzt. Nicht umgesetztes Kohlenmonoxid und ggf. Wasserstoff werden im Kreislauf geführt, während ein geringer Teilstrom das System als Abgas verlässt. Frisches, ggf. wasserstoffhaltiges Kohlenmonoxid wird dem Kreislaufgas entsprechend dem Umsatz zudosiert. Die dem Umsatz entsprechenden Mengen frisches Methylacetat und/oder Dimethylether sowie ggf. Methanol und/oder Wasser werden dem Reaktor zugeleitet. Im Masse der Aufgabe strömt das Reaktionsgemisch aus dem Reaktor ab. In einem Kurzwegverdampfer wird das Reaktionsgemisch von dem Katalysatorsystem, das dem Reaktor wieder zugeführt wird, abgetrennt. In einer ersten Kolonne erfolgt die Abtrennung der Leichtsieder (Methylacetat, Dimethylether, Methyljodid), die wiederum in den Reaktor gelangen. Der Sumpf der ersten Kolonne wird in zwei weiteren Kolonnen zu Essigsäure und Essigsäureanhydrid aufgearbeitet.

Beispiel 1

250 g Methylacetat, 1,6 g $RhCl_3 \cdot 3H_2O$, 60 g Methyljodid und 70 g Tetrabutylphosphoniumjodid werden in einem Hastelloy-Autoklaven vorgelegt; durch Aufdrücken von CO wird ein Druck von 25 bar eingestellt. Nach dem Aufheizen auf die Reaktionstemperatur von 455 K wird durch kontinuierliches Nachdrücken von CO 50 min lang ein Gesamtdruck von 50 bar aufrechterhalten. Nach dem Abkühlen und Entspannen werden durch gaschromatographische Analyse des Reaktionsgemisches 276 g Essigsäureanhydrid gefunden, entsprechend 1766 g $Ac_2O/g \, Rh \cdot h$.

Beispiel 2

200 g Dimethylether, 1,6 g $RhCl_3 \cdot 3H_2O$, 70 g Methyljodid und 80 g Tetrabutylphosphoniumjodid werden bei 455 K und 60 bar mit CO im Hastelloy-Autoklaven umgesetzt. Nach einer Reaktionszeit von 15 min werden im Reaktionsgemisch 228 g Essigsäureanhydrid und 156 g Methylacetat gefunden, entsprechend 1459 g $Ac_2O/g \, Rh \cdot h$.

Beispiel 3

300 g Methylacetat, 1,6 g $RhCl_3 \cdot 3H_2O$, 90 g Methyljodid, 70 g Essigsäure und 70 g Tetrabutylphosphoniumjodid werden in einem Autoklaven mit CO bei 50 bar und 453 K umgesetzt. Nach einer Reaktionszeit von 15 min werden 298 g Essigsäureanhydrid gefunden, entsprechend 1907 g $Ac_2O/g \, Rh \cdot h$.

Beispiel 4

280 g Methylacetat, 2 g $Pd(OAc)_2$, 70 g Methyljodid und 100 g Tetrabutylphosphoniumjodid werden in einem Hastelloy-Autoklaven bei 460 K und 50 bar mit CO umgesetzt. Nach 65 min werden 203 g Essigsäureanhydrid, entsprechend 198 g $Ac_2O/g \, Pd \cdot h$, erhalten.

Beispiel 5

250 g Methylacetat, 0,8 g $RhCl_3 \cdot 3H_2O$, 5 g Zirkoniumacetylacetonat, 90 g Methyljodid, 90 g Essigsäure und 120 g Tetrabutylphosphoniumjodid werden im Hastelloy-Autoklaven mit CO bei 70 bar und 458 K umgesetzt. Nach einer Reaktionszeit von 25 min werden 282 g Essigsäureanhydrid festgestellt, entsprechend 2165 g $Ac_2O/g \, Rh \cdot h$.

**Beispiel 6**

280 g Methylacetat, 1,8 g $RhCl_3 \cdot 3H_2O$, 8 g Vanadiumhexacarbonyl, 90 g Methyljodid und 70 g Tetrabutylphosphoniumjodid werden in einem Hastelloy-Autoklaven mit CO bei 50 bar und 460 K umgesetzt. Nach einer Reaktionszeit von 15 min werden 292 g Essigsäureanhydrid gefunden, entsprechend 1661 g $Ac_2O$/g Rh · h.

**Beispiel 7**

250 g Methylacetat, 1,6 g $RhCl_3 \cdot 3H_2O$, 7 g Chromhexacarbonyl, 80 g Methyljodid und 75 g Tetrabutylphosphoniumjodid werden bei 450 K und 60 bar in einem Hastelloy-Autoklaven mit CO umgesetzt. Nach 14 min werden 294 g Essigsäureanhydrid erhalten, entsprechend einer Leistung von 2016 g $Ac_2O$/g Rh · h.

**Beispiel 8**

250 g Methylacetat, 1,6 g $RhCl_3 \cdot 3H_2O$, 10 g Dirheniumdekacarbonyl, 150 g Methyljodid, 75 g Essigsäure und 100 g Tetrabutylphosphoniumjodid werden bei 453 K und 60 bar in einem Hastelloy-Autoklaven mit CO umgesetzt. Nach 15 min werden 286 g Essigsäureanhydrid im Reaktionsgemisch nachgewiesen. Das entspricht einer Leistung von 1830 g $Ac_2O$/g Rh · h.

**Beispiel 9**

280 g Methylacetat, 2 g $Pd(OAc)_2$, 10 g Dicobaltoctacarbonyl, 60 g Methyljodid und 100 g Tetrabutylphosphoniumjodid werden bei 458 K und 80 bar in einem Hastelloy-Autoklaven mit CO umgesetzt. Es werden nach 25 min 248 g Essigsäureanhydrid im Reaktionsgemisch ermittelt, entsprechend einer Leistung von 628 g $Ac_2O$/g Pd · h.

**Beispiel 10**

250 g Methylacetat, 50 g Methanol, 1,6 g $RhCl_3 \cdot 3H_2O$, 100 g Methyljodid und 70 g Tetrabutylphosphoniumjodid werden in einem Hastelloy-Autoklaven mit CO bei 50 bar und 455 K umgesetzt. Nach einer Reaktionszeit von 15 min werden 288 g Essigsäureanhydrid und 94 g Essigsäure erhalten, entsprechend einer Leistung von 1843 g $Ac_2O$/g Rh · h und 601 g Essigsäure/g Rh · h.

**Beispiel 11**

200 g Dimethylether, 50 g Methanol, 1,6 g $RhCl_3 \cdot 3H_2O$, 90 g Methyljodid und 90 g Tetrabutylphosphoniumjodid werden in einem Hastelloy-Autoklaven mit CO bei 60 bar und 458 K umgesetzt. Nach einer Reaktionszeit von 15 min werden 242 g Essigsäureanhydrid, 146 g Methylacetat und 94 g Essigsäure erhalten, entsprechend einer Leistung von 1549 g $Ac_2O$/g Rh · h und 601 g Essigsäure/g Rh · h.

**Beispiel 12**

300 g Dimethylether, 1,8 g $RhCl_3 \cdot 3H_2O$, 100 g Methyljodid, 100 g Essigsäure und 80 g Tetrabutylphosphoniumjodid werden in einem Hastelloy-Autoklaven bei 458 K und 80 bar mit einem Gemisch von CO und $H_2$, das 5 Vol% $H_2$ enthält, umgesetzt. Es werden nach 15 min 340 g Essigsäureanhydrid neben 160 g Methylacetat, 70 g Ethylidendiacetat und 30 g zusätzlich gebildeter Essigsäure ermittelt, entsprechend einer Leistung von 1935 g $Ac_2O$/g Rh · h und 171 g Essigsäure/g Rh · h.

**Beispiel 13**

200 g Methylacetat, 300 g Methanol, 1,6 g $RhCl_3 \cdot 3H_2O$, 100 g Methyljodid und 75 g Tetrabutylphosphoniumjodid werden in einem Hastelloy-Autoklaven mit CO bei 50 bar und 455 K umgesetzt. Nach einer Reaktionszeit von 18 min werden 220 g Essigsäureanhydrid und 208 g Essigsäure erhalten, entsprechend einer Leistung von 1173 g $Ac_2O$/g Rh · h und 1109 g Essigsäure pro g Rh · h.

**Beispiel 14**

300 g Methylacetat, 36 g Wasser, 70 g Jodwasserstoff, 1,6 g $RhCl_3 \cdot 3H_2O$ und 80 g Tetrabutylphosphoniumjodid werden in einem Hastelloy-Autoklaven bei 458 K und 40 bar mit CO umgesetzt. Nach einer Reaktionszeit von 14 min werden 122 g Essigsäureanhydrid und 273 g Essigsäure erhalten, entsprechend einer Leistung von 836 g $Ac_2O$/g Rh · h und 1872 g Essigsäure pro g Rh · h.

**Patentansprüche**

1. Verfahren zur Herstellung von Essigsäureanhydrid und ggf. Essigsäure durch Umsetzung von Kohlenmonoxid und ggf. Wasserstoff mit Methylacetat und/oder Dimethylether, ggf. in Mischung mit Methanol und/oder Wasser, bei Temperaturen von 350 bis 575 K und Drücken von 1 bis 300 bar in Gegenwart eines Katalysatorsystems aus Edelmetallen der Gruppe VIII des Periodensystems der Elemente oder deren Verbindungen, Jod und/oder dessen Verbindungen sowie ggf. carbonylbildenden Nichtedelmetallen der Gruppen IV, V, VI, VII oder VIII des Periodensystems der Elemente oder deren Verbindungen und ggf. einer aliphatischen Carbonsäure mit 1 bis 4 Kohlenstoffatomen, dadurch gekennzeichnet, dass das Katalysatorsystem als zusätzlichen Promotor Tetrabutylphosphoniumjodid enthält.

2. Verfahren nach Anspruch 1, dadurch gekennzeichnet, dass man das Katalysatorsystem Edelmetall(-verbindung)/Jod(-verbindung)/carbonylbildendes Nichtedelmetall(-verbindung)/ Carbonsäure/Tetrabutylphosphoniumjodid im Atom- bzw. Molverhältnis 1 : (1–1400) : (0–10) : (0–2000) : (1–1200) einsetzt.

3. Verfahren nach Anspruch 1 oder 2, dadurch gekennzeichnet, dass man Methylacetat, Methanol und Wasser oder Dimethylether, Methanol und Wasser im Molverhältnis 1 : (0 bis 5) : (0 bis 1) einsetzt.

4. Verfahren nach einem der Ansprüche 1–3, dadurch gekennzeichnet, dass man Kohlenmonoxid/Wasserstoffgemische mit bis zu 10 Vol% Wasserstoff einsetzt.

## Claims

1. A process for the preparation of acetic anhydride and possibly acetic acid by reaction of carbon monoxide and possibly hydrogen with methyl acetate and/or dimethylether, possibly as a mixture with methanol and/or water, at temperatures from 350 to 575 K and pressures from 1 to 300 bar in the presence of a catalyst system consisting of noble metals of group VIII of the periodic table of the elements or compounds thereof, iodine and/or compounds thereof and possibly carbonylforming base metals from groups IV, V, VI, VII or VIII of the periodic table of the elements or compounds thereof and possibly an aliphatic carboxylic acid having 1 to 4 carbon atoms, wherein the catalyst system contains tetrabutylphosphonium iodide as an additional promoter.

2. The process as claimed in claim 1, wherein the catalyst system noble metal (compound)/iodine (compound)/carbonyl-forming base metal (compound)/carboxylic acid/tetrabutylphosphonium iodide is used in an atomic or molar ratio of 1:(1–1400):(0–10):(0–2000):(1–1200).

3. The process as claimed in claim 1 or 2, wherein the methyl acetate, methanol and water or dimethyl ether, methanol and water are used in a molar ratio of 1:(0 to 5):(0 to 1).

4. The process as claimed in one of claims 1 to 3, wherein carbon monoxide/hydrogen mixtures containing up to 10% by volume of hydrogen are used.

## Revendications

1. Procédé de préparation de l'anhydride acétique et le cas échéant de l'acide acétique par réaction de l'oxyde de carbone et le cas échéant de l'hydrogène avec l'acétate de méthyle et/ou l'éther diméthylique, éventuellement en mélange avec du méthanol et/ou de l'eau, à des températures de 350 à 575° K et des pressions de 1 à 300 bars en présence d'un système catalyseur consistant en métaux nobles du groupe VIII de la Classification Périodique des Eléments ou leurs composés, l'iode et/ou ses composés et le cas échéant des métaux non nobles, formant des dérivés carbonyle, des groupes IV, V, VI, VII ou VIII de la Classification Périodique des Eléments ou leurs composés et le cas échéant un acide carboxylique aliphatique en C$_1$–C$_4$, caractérisé en ce que le système catalyseur contient en tant qu'activateur supplémentaire de l'iodure de tétrabutylphosphonium.

2. Procédé selon la revendication 1, caractérisé en ce que le système catalyseur métal noble (composé de)/iode (composé de)/métal non noble formant des dérivés carbonylés (composé de)/acide carboxylique/iodure de tétrabutylphosphonium est mis en œuvre dans des proportions atomiques ou moléculaires respectivement de 1:(1 à 1400):(0 à 10):(0 à 2000):(1 à 1200).

3. Procédé selon la revendication 1 ou 2, caractérisé en ce que l'acétate de méthyle, le méthanol et l'eau ou l'éther diméthylique, le méthanol et l'eau sont mis en œuvre dans des proportions molaires de 1:(0 à 5):(0 à 1).

4. Procédé selon l'une des revendications 1 à 3, caractérisé en ce que l'on met en œuvre des mélanges oxyde de carbone/hydrogène contenant jusqu'à 10% en volume de ce dernier.